# EUROPEAN PATENT APPLICATION

(11) **EP 4 717 167 A1**
(43) Date of publication of application: **01.04.2026**
(21) Application number: 25170209.8
(22) Date of filing: 11.04.2025
(51) Int. Cl.: A61B 5/145

(54) **AN INSERTING APPARATUS FOR SUBCUTANEOUSLY IMPLANTABLE BIOSENSOR PLACEMENT AND METHOD THEREOF**

(30) Priority: 30.09.2024 CN 202411391591; 06.03.2025 CN 202510265037
(71) Applicant: Jiangsu Yuwell Poctech Biotechnology Co., Ltd., Zhenjiang, Jiangsu 212300 (CN); Jiangsu Yuekai Biotechnology Co., Ltd., Nanjing, Jiangsu 210018 (CN); Zhejiang Poctech Co., Ltd., Huzhou, Zhejiang 313001 (CN)
(72) Inventor: WANG, Liang, Zhenjiang, Jiangsu, 212300 (CN); ZHANG, Yanan, Zhenjiang, Jiangsu, 212300 (CN)
(74) Representative: Dehns Germany Partnerschaft mbB

(57) **Abstract**

The present disclosure relates to an inserting apparatus for subcutaneously implantable biosensor placement and a method thereof. The inserting apparatus includes a mounting base, a fixing portion, a driving unit, a monitoring unit and a limiting unit. The mounting base is adapted to be attached to skin of a host. The fixing portion is disposed in a housing of the apparatus and detachably engaged with the mounting base. The driving unit is movably disposed in the fixing portion. The monitoring unit is detachably connected to the driving unit. The driving unit is configured to move relative to the fixing portion to drive the monitoring unit to be mounted at the mounting base. The limiting unit is configured to prevent the fixing portion from moving relative to the housing during mounting of the monitoring unit to the mounting base. A possibility of a movement of the mounting base relative to the fixing portion during mounting of the monitoring unit can be reduced, which can increase a success rate of implantation of the monitoring unit.

## Description

### FIELD

The present disclosure relates to the field of medical device technologies, and more particularly, to an inserting apparatus for subcutaneously implantable biosensor placement and a method thereof.

### BACKGROUND

Detecting various analytes in an individual's body is crucial for monitoring their health status. Deviations from normal analyte levels typically indicate potential physiological conditions, such as metabolic status or diseases. For many individuals, regular in vitro analyte monitoring using extracted body fluids is sufficient to observe their physiological status. However, in vitro analyte monitoring, due to the limited number of measurements, may miss critical measurement data, thereby causing a delay in optimal treatment timing and potentially causing irreversible harm to the patient. Additionally, for individuals with severe analyte imbalances and/or rapid fluctuations in analyte levels, frequent extraction of body fluids for monitoring is required, which can cause inconvenience and discomfort to the patient.

In many cases, subcutaneous, interstitial, or transdermal analyte sensors can provide adequate measurement accuracy while minimizing discomfort to the user. Continuous analyte monitoring using implanted analyte monitoring sensors is a more ideal monitoring method. Typically, an inserting apparatus is used to implant the analyte monitoring sensor into the individual's body. The sensor reacts with the body fluids of the host to generate electrical signals. The processing unit converts the electrical signals into data that can represent the analyte concentration, which is then transmitted to a display device for display, thereby achieving continuous monitoring of analyte concentration.

Each time a new sensor is used, the user inserts a portion of the sensor under the skin. Typically, an inserting apparatus is used to insert the sensor into the user's body. The puncture needle within the inserting apparatus is engaged with the sensor and carries it under the user's skin. After the sensor is implanted in place, the puncture needle is removed from the user' body while the sensor remains in the user's body. Existing inserting apparatuses, such as those disclosed in US9931065B2 and CN113827230B, have the technical problem that the implantation success rate is difficult to guarantee during the implantation process.

### SUMMARY

In view of this, the present disclosure provides an inserting apparatus for subcutaneously implantable biosensor placement and a method thereof, to increase a success rate of implantation of a monitoring unit.

In a first aspect, an inserting apparatus for subcutaneously implantable biosensor placement is provided according to an embodiment of the present disclosure. The inserting apparatus includes: a mounting base adapted to be attached to skin of a host; a fixing portion disposed in a housing of the inserting apparatus and removably engaged with the mounting base; a driving unit movably disposed in the fixing portion; a monitoring unit detachably connected to the driving unit, the driving unit being movable relative to the fixing portion to drive the monitoring unit to be mounted at the mounting base; and a limiting unit configured to prevent the fixing portion from moving relative to the housing during mounting of the monitoring unit at the mounting base.

In a possible embodiment, the limiting unit includes a first limit portion located on the fixing portion and a first engagement portion located on the mounting base, the first engagement portion being engaged with the first limit portion to prevent the fixing portion from moving relative to the mounting base.

In a possible embodiment, the driving unit is configured to move in a first direction to drive the monitoring unit to move from a first position to a second position. The monitoring unit is mounted at the mounting base at second position. Before the monitoring unit reaches the second position, the first limit portion is configured to be engaged with the first engagement portion to limit the relative movement between the fixing portion and the mounting base.

In a possible embodiment, at the second position, a force between the first limit portion and the first engagement portion is smaller than an adhesion force between the mounting base and the skin.

In a possible embodiment, at the second position, at least part of the first limit portion is disengaged from the first engagement portion to allow the fixing portion to move relative to the mounting base.

In a possible embodiment, the fixing portion has a mounting wall and a connection arm connected between the mounting wall and the first limit portion. The driving unit includes an abut portion, at the first position, the abut portion being spaced apart from the connection arm. When the driving unit moves in the first direction, the abut portion is capable of abutting with the connection arm to drive the connection arm to move or deform in a direction gradually away from the first engagement portion.

In a possible embodiment, an acute angle between the connection arm and the first direction ranges from 15° to 45°.

In a possible embodiment, the driving unit includes an insertion assembly and a first driver, the insertion assembly including at least one second limit portion. The fixing portion includes at least one second engagement portion. The at least one second limit portion is engaged with the at least one second engagement portion to restrict a movement of the insertion assembly relative to the fixing portion, the at least one second limit portion being configured to be moveable or deformable relative to the fixing portion, to allow the driving unit to move relative to the fixing portion in the first direction.

In a possible embodiment, the inserting apparatus further includes a trigger, the trigger being adapted to abut against the second limit portion to make the second limit portion move or deform relative to the fixing portion, releasing an engagement between the second limit portion and the second engagement portion.

In a possible embodiment, the inserting apparatus further includes a locking member for preventing the trigger from abutting against the second limit portion.

In a possible embodiment, the mounting base includes a main body and a fifth limit portion, the fifth limit portion being swingable relative to the main body. The monitoring unit includes a fifth engagement portion. The fifth limit portion is engaged with the fifth engagement portion to restrict the monitoring unit from detaching from the mounting base.

The present disclosure further provides a method of using an inserting apparatus. The inserting apparatus includes a fixing portion, a driving unit, a monitoring unit, and a mounting base. The method includes: driving, by the driving unit, the monitoring unit to move from a first position relative to the fixing portion in a first direction, wherein at the first position, a first limit portion of the fixing portion is engaged with a first engagement portion of the mounting base to prevent the mounting base from moving relative to the fixing portion; driving, by the driving unit, the first limit portion to gradually move or deform in a direction away from the first engagement portion; and moving the driving unit to a second position, and mounting the monitoring unit to the mounting base, wherein at the second position, an engagement between the first limit portion and the first engagement portion is at least partially released.

In a possible embodiment, at the second position, the first limit portion and the first engagement portion are completely disengaged from each other.

In a possible embodiment, the inserting apparatus further includes a trigger, the driving unit includes a second limit portion, and the fixing portion includes an avoidance space and a second engagement portion disposed in the avoidance space. The method further includes: driving, by the trigger, the second limit portion to move or deform, to disengage the second limit portion from the second engagement portion, enabling the driving unit to drive the monitoring unit to move in the first direction.

In a possible embodiment, the mounting base includes a fifth limit portion, and the monitoring unit includes a fifth engagement portion. The method further includes: driving, by the fifth engagement portion, the fifth limit portion to move or deform in a process of the driving unit driving the monitoring unit to move in the first direction; and restoring, in response to the monitoring unit reaching the second position, the fifth limit portion, and engaging the fifth limit portion with the fifth engagement portion to prevent the monitoring unit from detaching from the mounting base.

According to the embodiments of the present disclosure, the inserting apparatus for subcutaneously implantable biosensor placement and the method thereof are provided. The limiting unit of the inserting apparatus is configured to prevent the mounting base from moving relative to the housing during mounting of the monitoring unit to the mounting base, to restrict a relative position between the fixing portion and the mounting base before the monitoring unit is mounted in place. Therefore, a possibility of a movement between the fixing portion and the mounting base is reduced, which in turn reduces a possibility of an implantation failure of the monitoring unit, facilitating an increase in the success rate of the implantation of the monitoring unit.

### BRIEF DESCRIPTION OF THE DRAWINGS

In order to clearly explain technical solutions of embodiments of the present disclosure, drawings used in the embodiments of the present disclosure are briefly described below. Obviously, the drawings as described below are merely some embodiments of the present disclosure. Based on these drawings, other drawings can be obtained by those skilled in the art without creative effort.
FIG. 1 is a schematic view of an inserting apparatus provided by the present disclosure.
FIG. 2 is a schematic internal view of an inserting apparatus provided by the present disclosure.
FIG. 3 is an enlarged partial view of part I in FIG. 2, in which a driving unit is located at a first position.
FIG. 4 is an enlarged partial view of part I in FIG. 2, in which a first engagement portion has a third contact surface and a driving unit is located between a first position and a second position.
FIG. 5 is an enlarged partial view of part I in FIG. 2, in which a first engagement portion has a third contact surface and a driving unit is located at a second position.
FIG. 6 is an enlarged partial view of part I in FIG. 2, in which a first engagement portion has a third contact surface and a fourth contact surface and a driving unit is located between a first position and a second position.
FIG. 7 is an enlarged partial view of part I in FIG. 2, in which a first engagement portion has a third contact surface and a fourth contact surface and a driving unit is located at a second position.
FIG. 8 is a front view of a driving unit provided by the present disclosure, in which the driving unit is located at a first position and a second limit portion is engaged with a second engagement portion.
FIG. 9 is a front view of a driving unit provided by the present disclosure, in which the driving unit is located at a first position and a second limit portion is disengaged from a second engagement portion.
FIG. 10 is a schematic view of a driving unit located between a first position and a second position provided by the present disclosure.
FIG. 11 is a side view of a driving unit located at a first position provided by the present disclosure.
FIG. 12 is a side view of a driving unit located at a second position provided by the present disclosure.
FIG. 13 is a side view of a monitoring unit reaching a second position and a retraction assembly being retracted provided by the present disclosure.
FIG. 14 is a schematic view of a locking member provided by the present disclosure.
FIG. 15 is a schematic internal view of an inserting apparatus including a locking member provided by the present disclosure.
FIG. 16 is a cross-sectional view of an inserting apparatus provided by the present disclosure.
FIG. 17 is a cross-sectional view of a fixing portion engaged with a driving unit according to a first embodiment provided by the present disclosure.
FIG. 18 is a schematic perspective view of a fixing portion according to a first embodiment provided by the present disclosure.
FIG. 19 is a schematic perspective view of a driving unit according to a first embodiment provided by the present disclosure.
FIG. 20 is a top view of a fixing portion engaged with a driving unit according to a second embodiment provided by the present disclosure.
FIG. 21 is a schematic partial view of a fixing portion according to a second embodiment provided by the present disclosure.
FIG. 22 is a top view of an insertion assembly engaged with a retraction assembly according to a second embodiment provided by the present disclosure.

Reference numerals of the accompanying drawings:
1-fixing portion; 11-first limit portion; 111-first limit segment; 111a-first contact surface; 112-second limit segment; 112a-second contact surface; 113-connection arm; 12-second engagement portion; 13-fourth engagement portion; 14-first guide portion; 16-third guide portion; 2-driving unit; 21-insertion assembly; 211-abut portion; 212-second limit portion; 213-third limit portion; 214-fourth limit portion; 215-second guide portion; 215b-raised portion; 216-fourth guide portion; 217-sixth guide portion; 22-retraction assembly; 221-third engagement portion; 222-fifth guide portion; 23-second driver; 3-monitoring unit; 31-fifth engagement portion; 4-mounting base; 41-first engagement portion; 411-third contact surface; 412-fourth contact surface; 42-main body; 43-fifth limit portion; 5-first driver; 6-locking member; 61-body portion; 62-first extension portion; 621-limit groove; 63-second extension portion; 7-trigger; 8-housing; 9-guide segment.

### DETAILED DESCRIPTION

To better understand technical solutions of the present disclosure, embodiments of the present disclosure are described in detail below in conjunction with the accompanying drawings.

It should be noted that, the embodiments described below are only a part of the embodiments of the present disclosure, rather than all of the embodiments. On a basis of the embodiments in the present disclosure, all other embodiments obtained by those skilled in the art without creative labor shall fall within the protection scope of the present disclosure.

Terms used in the embodiments of the present disclosure are only for a purpose of describing specific embodiments, and are not intended to limit the present disclosure. Singular forms of "a", "said", and "the" used in the embodiments of the present disclosure and the claims as attached also include plural forms, unless the context clearly indicates other meanings.

It should be understood that the term "and/or" as used herein only represents a relationship between correlated objects, including three relationships. For example, "A and/or B" may mean three situations: A only, B only, or both A and B. In addition, the character "/" in the present disclosure generally represents an "or" relationship between the correlated objects preceding and succeeding the symbol.

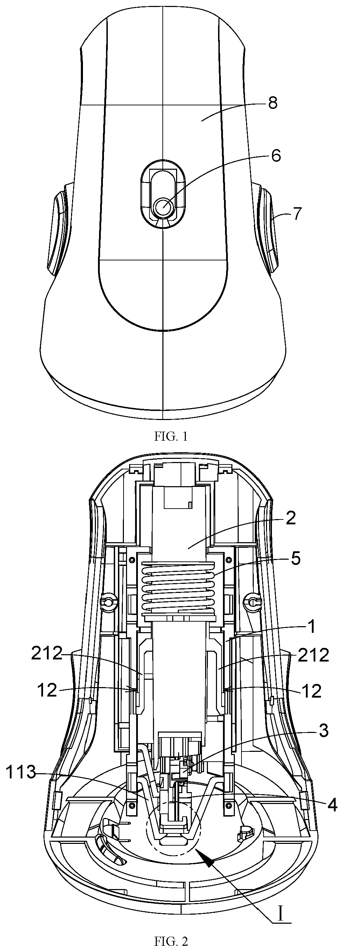

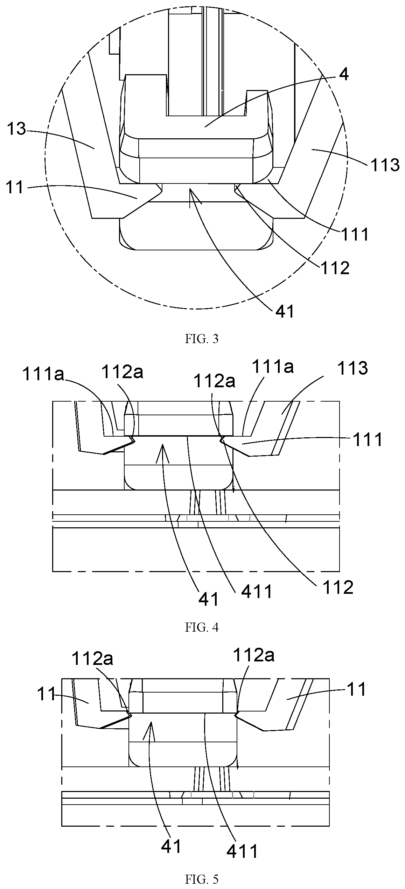

As illustrated in FIG. 1, an inserting apparatus for subcutaneously implantable biosensor placement is provided according to an embodiment of the present disclosure. A sensor can be implanted into a body of a subject to facilitate monitoring of glucose of the subject. As illustrated in FIG. 2, the inserting apparatus includes a fixing portion 1, a driving unit 2, a monitoring unit 3, a mounting base 4 and a housing 8. The fixing portion 1 may be disposed in a housing 8 of the inserting apparatus. The driving unit 2 is slidably mounted in the fixing portion 1. The monitoring unit 3 is detachably mounted at the driving unit 2. The driving unit 2 is movable in a first direction relative to the fixing portion 1 and is capable of driving the monitoring unit 3 to move from a first position to a second position in the first direction. The mounting base 4 is configured to accommodate the monitoring unit 3. As illustrated in FIG. 3, the fixing portion 1 is provided with at least two first limit portions 11. The mounting base 4 is provided with a first engagement portion 41. The first engagement portion 41 is configured to form a limiting unit together with a first limit portion 11. The limiting unit is configured to prevent the mounting base 4 from moving relative to the housing 8 during mounting of the monitoring unit 3 to the mounting base 4. Specifically, at the first position, the first limit portion 11 is engaged with the first engagement portion 41, for restricting a movement of the mounting base 4 relative to the fixing portion 1. At the second position, the monitoring unit 3 is mounted at the mounting base 4. Before the monitoring unit 3 reaches the second position, the first limit portion 11 is capable of preventing the mounting base 4 from moving relative to the fixing portion 1. That is, before the monitoring unit 3 is mounted at the mounting base 4, the first limit portion 11 may be in an engagement state with the first engagement portion 41, in such a manner that a relative movement between the mounting base 4 and the fixing portion 1 can be restricted.

In a solution according to an embodiment of the present disclosure, with a positional constraint achieved through a limiting engagement between the first limit portion 11 and the first engagement portion 41, a relative position between the mounting base 4 and the fixing portion 1 can be restricted during mounting of the monitoring unit 3 at the mounting base 4. Therefore, a possibility of a movement of the mounting base 4 relative to the fixing portion 1 during the mounting of the monitoring unit 3 can be reduced, which avoids a failure of the mounting of the monitoring unit 3 due to the relative movement occurred between the fixing portion 1 and the mounting base 4 before the monitoring unit 3 is mounted in place, facilitating increasing a success rate of implantation of the monitoring unit 3.

Since the monitoring unit 3 needs to be implanted in the human body, the skin will be punctured during the implantation. Therefore, when the mounting base 4 shakes during the implantation, the monitoring unit 3 is driven to shake, which is likely to lead to a larger wound area in the human body and therefore aggravates pain and bleeding, compromising use experience of a user. With the solution according to the embodiment of the present disclosure, the first limit portion 11 and the first engagement portion 41 remain in the engagement state before the monitoring unit 3 is mounted at the mounting base 4, in such a manner that a position of the mounting base 4 is restricted to reduce a possibility of shaking or the like of the mounting base 4, which can reduce a possibility of shaking of the mounting base 4 during the implantation. Therefore, a reduction of the wound area can be facilitated to alleviate the pain and the bleeding during the implantation, which is conductive to improving the use experience of the user and is more in line with an actual use demand.

The mounting base 4 may be disposed at a surface of the skin by means of adhesion or the like and fixed relative to the skin, to prevent the mounting base 4 and the monitoring unit 3 mounted at the mounting base 4 from falling off from the human body. When located at the second position, the monitoring unit 3 has been mounted at the mounting base 4, and the implantation has been completed. After the implantation is completed, the fixing portion 1 needs to be separated from the mounting base 4. In this case, the first limit portion 11 may be in a state of being completely or partially disengaged with the mounting base 4. When the first limit portion 11 is completely disengaged with the mounting base 4, no force is assumed to act between the first limit portion 11 and the mounting base 4. Due to an adhesion force between the mounting base 4 and the skin, only the fixing portion 1 is removed when the fixing portion 1 is separated from the mounting base 4, leaving the mounting base 4 and the monitoring unit 3 mounted at the mounting base 4 in the human body. When the first limit portion 11 is partially disengaged with the mounting base 4, a certain force still exists between the first limit portion 11 and the mounting base 4, for restricting the fixing portion 1 from being separated from the mounting base 4. To remove the fixing portion 1, the user needs to apply an external force to the fixing portion 1 to counteract the force acting between the fixing portion 1 and the mounting base 4. In the solution according to the embodiment of the present disclosure, at the second position, the force acting between the fixing portion 1 and the mounting base 4 is smaller than the adhesion force between the mounting base 4 and the skin, which can reduce a possibility of disengaging the mounting base 4 from the skin in a process of removing the fixing portion 1.

In a possible embodiment, at the second position, at least part of the first limit portion 11 is disengaged with the first engagement portion 41. In this way, after the monitoring unit 3 is mounted in place, removal of a structure such as the fixing portion 1 can be facilitated for leaving the mounting base 4 and the monitoring unit 3 in the human body.

At the second position, the first limit portion 11 may be completely disengaged with the first engagement portion 41 for ease of separation of the fixing portion 1 and the mounting base 4, which facilitates an operation of the user. The first limit portion 11 may also be partially disengaged from and partially engaged with the first engagement portion 41 to retain a certain level of positional constraint between the fixing portion 1 and the mounting base 4, which can reduce a possibility of a relative movement between the fixing portion 1 and the mounting base 4 before the user removes the fixing portion 1. When the user considers it necessary to remove the fixing portion 1, the external force is applied to separate the fixing portion 1 from the mounting base 4. In addition, since the first limit portion 11 remains partially engaged with the first engagement portion 41 after the monitoring unit 3 is mounted in place, stability of the mounting base 4 can be further improved, which is conducive to reducing the possibility of shaking, movement, or the like of the mounting base 4 before the monitoring unit 3 is mounted in place.

As illustrated in FIG. 3, in a possible embodiment, the first limit portion 11 includes a first limit segment 111 and a second limit segment 112 connected to the first limit segment 111. An obtuse angle is formed between the first limit segment 111 and the second limit segment 112. Before the monitoring unit 3 reaches the second position, the first limit segment 111 cooperates with the first limit portion 11. When the monitoring unit 3 reaches the second position, the second limit segment 112 cooperates with the first limit portion 11.

The first limit segment 111 and the second limit segment 112 may be engaged with the first limit portion 11 at different stages to change the engagement state between the first limit portion 11 and the first engagement portion 41. An angle between the first limit segment 111 and the second limit segment 112 can enable an engagement state between the first limit segment 111 and the first engagement portion 41 to be different from that between the second limit segment 112 and the first engagement portion 41, including but not limited to different contact areas during an engagement (increasing differences in magnitudes of stressing forces). By changing the engagement state between the first limit portion 11 and the first engagement portion 41, the fixing portion 1 can apply varying degrees of positional constraint on the mounting base 4. Before the monitoring unit 3 is mounted at the mounting base 4, the first limit portion 11 applies a tighter constraint on the mounting base 4 to prevent the mounting base 4 from shaking, vibrating, and the like during the mounting of the monitoring unit 3. Therefore, the movement of the mounting base 4 can be avoided to prevent the success rate of the implantation of the monitoring unit 3 from being affected. After the monitoring unit 3 is mounted at the mounting base 4, the first limit portion 11 partially releases the constraint on the mounting base 4, which reduces a difficulty for the user to remove the fixing portion 1 after the implantation is completed, saving effort and facilitating an operation.

As illustrated in FIG. 4, in a possible embodiment, the first limit segment 111 includes a first contact surface 111a. The second limit segment 112 includes a second contact surface 112a. An acute angle α is formed between the first contact surface 111a and the second contact surface 112a, where 0°<α< 90°. That is, the first contact surface 111a is neither parallel nor perpendicular to the second contact surface 112a. In a direction away from the first contact surface 111a, the second contact surface 112a may be inclined away from the driving unit 2. The first engagement portion 41 includes a third contact surface 411. As illustrated in FIG. 4, when the monitoring unit 3 is located at the first position, the first contact surface 111a abuts with the third contact surface 411. As illustrated in FIG. 5, when the monitoring unit 3 is located at the second position, the second contact surface 112a abuts with the third contact surface 411.

Due to different angles of the first contact surface 111a and the second contact surface 112a, a contact area between the first contact surface 111a and the third contact surface 411 when the first contact surface 111a and the third contact surface 411 are engaged with each other is different from that between the second contact surface 112a and the third contact surface 411 when the second contact surface 112a and the third contact surface 411 are engaged with each other, in such a manner that the first limit portion 11 and the first engagement portion 41 are in different engagement states. When the first limit portion 11 and the first engagement portion 41 are in the different engagement states, a level of constraint applied by the first limit portion 11 on the mounting base 4 varies. Before the monitoring unit 3 is mounted in place, the first limit portion 11 applies a larger constraint on the mounting base 4 to restrict the relative movement between the mounting base 4 and the fixing portion 1, favorably increasing the success rate of the implantation of the monitoring unit 3. After the monitoring unit 3 is mounted in place, the first limit portion 11 applies a smaller constraint on the mounting base 4, and thus it is easier for the user to separate the fixing portion 1 from the mounting base 4, making the operation simpler and less laborious.

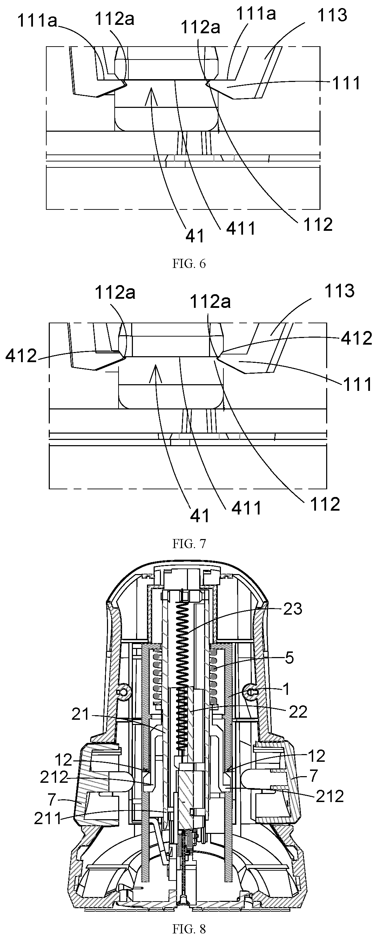

As illustrated in FIG. 6 and FIG. 7, in a possible embodiment, when the monitoring unit 3 is located at the first position, the first limit portion 11 abuts with the third contact surface 411 through the first contact surface 111a. In this case, the first contact surface 111a and the third contact surface 411 are in a parallel state, and thus a large contact area exists between the first contact surface 111a and the third contact surface 411. An angle may be formed between each of the first contact surface 111a and the third contact surface 411 and the first direction. The angle is related to an implantation angle for the inserting apparatus. Typically, the angle between each of the first contact surface 111a and the third contact surface 411 and the first direction may be identical to the implantation angle, or vary within a certain range relative to the implantation angle. Typically, each of the first contact surface 111a and the third contact surface 411 may be parallel to a top surface of the mounting base 4. The top surface of the mounting base 4 is at a side of the mounting base 4 away from the human body. With such a design, the fixing portion 1 can be restricted from moving away from the human body relative to the mounting base 4 when the first contact surface 111a is engaged with the third contact surface 411, which can prevent the relative movement between the fixing portion 1 and the mounting base 4 before the monitoring unit 3 is mounted in place, further preventing the fixing portion 1 from leaving the mounting base 4. When the monitoring unit 3 is located at the second position, the first limit portion 11 is in contact with the third contact surface 411 through the second contact surface 112a. In this case, instead of being parallel to the third contact surface 411, the second contact surface 112a is inclined relative to the third contact surface 411, and a relatively small contact area exists between the second contact surface 112a and the third contact surface 411. In a possible embodiment, the second contact surface 112a is gradually inclined towards an interior of the fixing portion 1 in the first direction. The second contact surface 112a may be used to form an inclined guide surface. When the second contact surface 112a is engaged with the first engagement portion 41, the first engagement portion 41 is slidable along the second contact surface 112a relative to the first limit portion 11, to disengage the first limit portion 11 from the first engagement portion 41. Since a contact area between the first limit portion 11 and the third contact surface 411 varies depending on a position of the monitoring unit 3, the level of constraint applied by the first limit portion 11 on the mounting base 4 also varies. A contact area between the first limit portion 11 and the mounting base 4 before the monitoring unit 3 is mounted at the mounting base 4 may be greater than that between the first limit portion 11 and the mounting base 4 after the monitoring unit 3 is mounted at the mounting base 4. Therefore, a possibility of shaking of the mounting base 4 relative to the fixing portion 1 before the monitoring unit 3 is mounted in place can be reduced, which can increase the success rate of the implantation of the monitoring unit 3. In addition, the reduction of the wound area can be facilitated to alleviate the pain, improving the use experience of the user.

In a possible embodiment, the first limit segment 111 includes the first contact surface 111a. The second limit segment 112 includes the second contact surface 112a. The first engagement portion 41 includes the third contact surface 411 and a fourth contact surface 412. When the monitoring unit 3 is located at the first position, the first contact surface 111a abuts with the third contact surface 411. The first contact surface 111a may be parallel to the third contact surface 411. When the monitoring unit 3 is located at the second position, the second contact surface 112a abuts with the fourth contact surface 412. The second contact surface 112a may be parallel to the fourth contact surface 412. To facilitate the separation of the fixing portion 1 and the mounting base 4 after the monitoring unit 3 is mounted, the second contact surface 112a and the fourth contact surface 412 can be used to form guide surfaces. The second contact surface 112a and the fourth contact surface 412 may be inclined relative to a direction of movement of the fixing portion 1 when the fixing portion 1 is removed. When the fixing portion 1 is removed, the second contact surface 112a and the fourth contact surface 412 can slide relative to each other to facilitate the separation of the fixing portion 1 and the mounting base 4.

As illustrated in FIG. 2, in a possible embodiment, the fixing portion 1 further includes a mounting wall and a connection arm 113 connected to the mounting wall and the first limit portion 11. The mounting wall may be an inner wall of the fixing portion 1. An acute angle is formed between an extension direction of the connection arm 113 and the first direction.

The connection arm 113 may be a structure such as a resilient arm. An adjustment of the position of the first limit portion 11 can be facilitated by setting the resilient arm. The position of the first limit portion 11 can be adjusted through changing the structure of the connection arm 113, an angle between the connection arm 113 and the first direction, or the like, in such a manner that the position of the first limit portion 11 can be set more flexibly, which is conducive to optimizing an internal structure of the inserting apparatus, making an overall structure more compact. In addition, through driving the connection arm 113, the first limit portion 11 may be driven to move relative to the first engagement portion 41, which is conducive to reducing a volume of the first limit portion 11 and facilitates driving the first limit portion 11.

In a possible embodiment, the angle between the connection arm 113 and the first direction ranges from 15° to 45°. The angle between the connection arm 113 and the first direction can be set as desired, including but not limited to 15°, 17°, 19°, 21°, 23°, 25°, 27°, 29°, 31°, 33°, 35°, 37°, 39°, 41°, 43°, 45°, and the like.

When the angle between the connection arm 113 and the first direction is too large, driving the connection arm 113 to drive the first limit portion 11 to be disengaged from the first engagement portion 41 is laborious. When the angle between the connection arm 113 and the first direction is too small, stability of the engagement between the first limit portion 11 and the first engagement portion 41 is likely to be reduced, which leads to accidental triggering during use, causing situations such as the first limit portion 11 being disengaged from the first engagement portion 41 prematurely. Such a situation results in a release of a limiting engagement between the mounting base 4 and the fixing portion 1 before the monitoring unit 3 reaches the second position, which leads to a relative movement, causing an implantation failure or the like. Therefore, the angle between the connection arm 113 and the first direction may be set to range from 15° to 45°. By taking into account factors such as a structure and a size of the inserting apparatus, the angle between the connection arm 113 and the first direction can be set to range from 15° to 23°, which not only enables the first limit portion 11 to be stably engaged with the first engagement portion 41, but also facilitates driving the connection arm 113 to drive the first limit portion 11 to move relative to the first engagement portion 41 for releasing a positional constraint. Such a design makes operations more effortless and convenient while contributing to a more compact structure of the inserting apparatus, which is conducive to a miniaturized design.

As illustrated in FIG. 8, in a possible embodiment, the driving unit 2 includes an abut portion 211. At the first position, the abut portion 211 is spaced apart from the connection arm 113. A spacing is available between the abut portion 211 and the connection arm 113 in the first direction. When the driving unit 2 moves in the first direction, the abut portion 211 is capable of being moved to abut with the connection arm 113, for driving the connection arm 113 to move or deform in a direction that is gradually away from the first engagement portion 41, driving the first limit portion 11 to be gradually disengaged from the first engagement portion 41.

By spacing the abut portion 211 apart from the connection arm 113, the driving unit 2 can be moved by a certain distance relative to the fixing portion 1, and then the driving unit 2 can come into contact with the connection arm 113 and drive the connection arm 113 to drive the first limit portion 11 to move, in such a manner that delayed driving of the connection arm 113 can be realized through a mechanical structure. That is, after an implantation action has begun for a period of time, the first limit portion 11 starts to be gradually disengaged from the first engagement portion 41. Such a design allows the implantation action to start out of synchronization with an action for releasing the engagement between the first limit portion 11 and the first engagement portion 41. By delaying driving the first limit portion 11 to be disengaged from the first engagement portion 41, a possibility of the fixing portion 1 being disengaged from the mounting base 4 before the monitoring unit 3 is mounted in place can be reduced, which can reduce the possibility of shaking, movement, or the like of the mounting base 4 due to an early release of the positional constraint on the mounting base 4. Therefore, the success rate of the implantation of the monitoring unit 3 can be increased, which is conductive to reducing the wound area to alleviate the pain and the bleeding, improving the use experience of the user.

As illustrated in FIG. 8, in a possible embodiment, the driving unit 2 includes an insertion assembly 21 and a first driver 5. The insertion assembly 21 includes at least one second limit portion 212. The fixing portion 1 includes at least one second engagement portion 12. An engagement between the second limit portion 212 and the second engagement portion 12 can be used to restrict a movement of the insertion assembly 21 relative to the fixing portion 1. The second limit portion 212 is movable or deformable relative to the fixing portion 1 to be disengaged from the second engagement portion 12, in such a manner that the insertion assembly 21 is movable relative to the fixing portion 1, which enables the driving unit 2 to move relative to the fixing portion 1 and perform the implantation action.

With the second limit portion 212 and the second engagement portion 12, a movement of the driving unit 2 can be controlled for controlling the implantation action. When the implantation action needs to be performed, a positional constraint on the driving unit 2 can be released through a release of the engagement between the second limit portion 212 and the second engagement portion 12, in such a manner that the driving unit 2 can move in the first direction to perform the implantation action.

In a possible embodiment, the first driver 5 may be an elastic member such as a spring. The first driver 5 may be in a deformed state before the second limit portion 212 is disengaged from the second engagement portion 12. After the first limit portion 11 is disengaged from the second engagement portion 12, a restoring force of the first driver 5 acts on the driving unit 2 to drive the driving unit 2 to move in the first direction.

A positional constraint between the driving unit 2 and the fixing portion 1 is realized through the engagement between the second limit portion 212 and the second engagement portion 12. A positional constraint between the fixing portion 1 and the mounting base 4 is realized through the engagement between the first limit portion 11 and the first engagement portion 41. When the implantation action needs to be performed, the engagement between the second limit portion 212 and the second engagement portion 12 is released, in such a manner that the driving unit 2 can move in the first direction for performing the implantation action. When the driving unit 2 moves until the abut portion 211 is in contact with the connection arm 113, the driving unit 2 continues to move in the first direction, for driving the connection arm 113 to swing to drive the first limit portion 11 to move away from the first engagement portion 41. That is, in the solution according to the embodiment of the present disclosure, the positional constraint on the driving unit 2 and the positional constraint on the mounting base 4 are not released simultaneously. When the engagement between the second limit portion 212 and the second engagement portion 12 is released, the engagement between the first limit portion 11 and the first engagement portion 41 is not released simultaneously or directly. Instead, the engagement between the first limit portion 11 and the first engagement portion 41 starts to be released after the implementation unit 2 moves until the abut portion 211 is in contact with the connection arm 113.

Such a design can prevent the positional constraint on the mounting base 4 to be simultaneously released when the positional constraint on the driving unit 2 is released. Therefore, during the implantation, a relative position between the mounting base 4 and the fixing portion 1 can still be restricted through the engagement between the first limit portion 11 and the first engagement portion 41. Therefore, the possibility of shaking or the like of the mounting base 4 during the implantation is reduced, which is conducive to improving the success rate of the implantation and is more in line with the actual use demand.

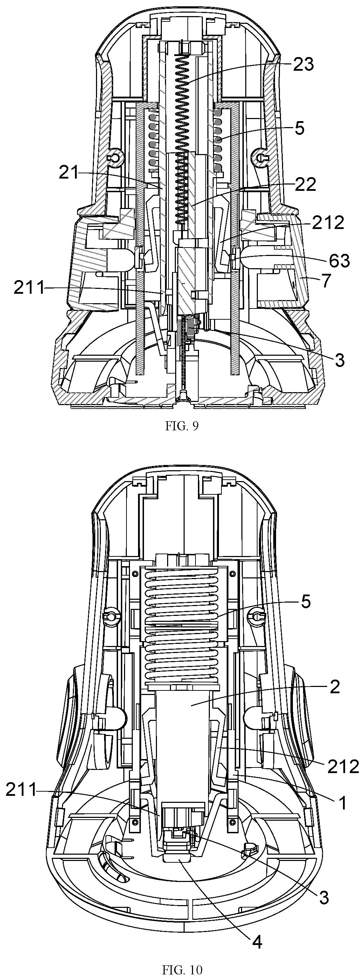

A state of the second limit portion 212 and the second engagement portion 12 after the second limit portion 212 is unlocked from the second engagement portion 12 is illustrated in FIG. 9. When the driving unit 2 drives the monitoring unit 3 to move to the second position, a state of the inserting apparatus is illustrated in FIG. 10.

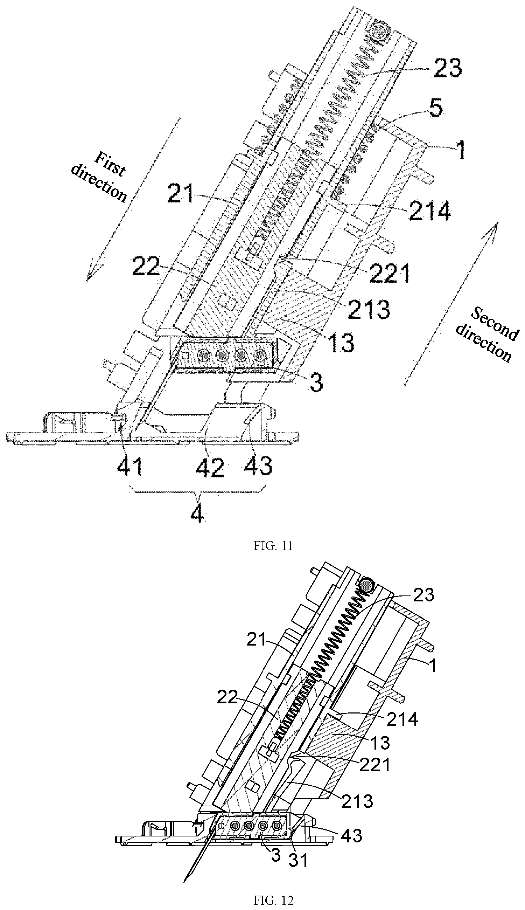

As illustrated in FIG. 11, FIG. 12, and FIG. 13, in a possible embodiment, the driving unit 2 further includes a retraction assembly 22 and a second driver 23. The retraction assembly 22 may be a half-wrapped needle assembly, etc. The insertion assembly 21 includes a third limit portion 213. The retraction assembly 22 includes a third engagement portion 221. The third limit portion 213 is engaged with the third engagement portion 221 to restrict the retraction assembly 22 from moving in a second direction relative to the insertion assembly 21. The first direction is opposite to the second direction. Normally, the first direction is a direction near the human body and the second direction is a direction away from the human body.

During the implantation action, the third limit portion 213 is engaged with the third engagement portion 221 to restrict a relative movement between the retraction assembly 22 and the insertion assembly 21, in such a manner that the retraction assembly 22 can move with the insertion assembly 21 in the first direction to perform the implantation action. The retraction assembly 22 is configured to pierce the skin to implant an electrode of the monitoring unit 3 inside the human body. After the monitoring unit 3 reaches the second position, i.e., after the monitoring unit 3 is mounted in place, the third limit portion 213 can be disengaged from the third engagement portion 221 to enable the second driver 23 to drive the retraction assembly 22 to move in the second direction opposite to the first direction. That is, the retraction assembly 22 performs a needle withdrawal action away from the human body after the implantation is completed.

As illustrated in FIG. 11, FIG. 12, and FIG. 13, in a possible embodiment, the fixing portion 1 includes a fourth engagement portion 13. During a movement of the driving unit 2 in the first direction, the fourth engagement portion 13 abuts with the third limit portion 213 for restricting the third limit portion 213 from detaching from the third engagement portion 221.

The fourth engagement portion 13 may be located at a side of the third limit portion 213 away from the third engagement portion 221, for restricting the third limit portion 213 from moving away from the third engagement portion 221, which therefore restricts the third limit portion 213 from detaching from the third engagement portion 221. The fourth engagement portion 13 is in contact with the third limit portion 213 both when the monitoring unit 3 is located at the first position and when the monitoring unit 3 moves from the first position to the second position. When the monitoring unit 3 is located at the second position, the mounting of the monitoring unit 3 is completed. The implantation assembly is moved until the fourth engagement portion 13 is disengaged from the third limit portion 213 and the third limit portion 213 is disengaged from the third engagement portion 221, in such a manner that the retraction assembly 22 is moveable relative to the insertion assembly 21 in the second direction to realize the needle withdrawal action.

With such a design, by using positional relationships between various components, the implantation action and the needle withdrawal action are controlled through the mechanical structure to realize automatic needle withdrawal, which is simple and less difficult to operate when in use and offers greater convenience for the user.

The second driver 23 may be an elastic member such as a spring. The second driver 23 has an end connected to the insertion assembly 21 and another end connected to the retraction assembly 22. The third limit portion 213 may be a structure such as a resilient arm. Before the third limit portion 213 is disengaged from the third engagement portion 221, the third limit portion 213 may be deformed to enable the third limit portion 213 to be engaged with the third engagement portion 221. In addition, the second driver 23 may be in a deformed state. The retraction assembly 22 may move with the insertion assembly 21 in the first direction to pierce the skin, which enables the electrode of the monitoring unit 3 to be implanted in the human body. After the mounting of the monitoring unit 3 is completed, the third limit portion 213 can recover from a deformation and be disengaged from the third engagement portion 221 since an abutment between the fourth engagement portion 13 and the third limit portion 213 is released. Therefore, a positional constraint between the insertion assembly 21 and the retraction assembly 22 is released. A restoring force of the second driver 23 can act on the retraction assembly 22 to drive the retraction assembly 22 to move relative to the insertion assembly 21 in the second direction, realizing the needle withdrawal action.

As illustrated in FIG. 11, FIG. 12, and FIG. 13, in a possible embodiment, the insertion assembly 21 includes a fourth limit portion 214. The fourth limit portion 214 protrudes in a direction close to the fixing portion 1. The fourth engagement portion 13 protrudes in a direction close to the insertion assembly 21. The fourth limit portion 214 is configured to be engaged with the fourth engagement portion, for restricting a movement distance of the driving unit 2 in the first direction.

When the monitoring unit 3 is located at the first position, the fourth limit portion 214 and the fourth engagement portion 13 have a spacing in the first direction. As the driving unit 2 moves in the first direction, the spacing between the fourth limit portion 214 and the fourth engagement portion 13 gradually decreases. When the monitoring unit 3 is located at the second position, the fourth limit portion 214 abuts with the fourth engagement portion 13 in the first direction for restricting the driving unit 2 from continuing to move in the first direction. Therefore, the movement distance of the driving unit 2 during the implantation action can be controlled to cause the driving unit 2 to drive the monitoring unit 3 to move in a predetermined range of movement, which avoids a possible mounting failure of the monitoring unit 3 due to a large movement distance of the driving unit 2 in the first direction. In addition, a distance at which the retraction assembly 22 is inserted into the human body can be controlled to prevent the retraction assembly 22 from being inserted into the human body at too large a distance, which aggravates the pain, affecting the use experience of the user.

As illustrated in FIG. 8, FIG. 9, and FIG. 10, in a possible embodiment, the inserting apparatus further includes a trigger 7. The fixing portion 1 further has an avoidance space. The trigger 7 passes through the avoidance space to abut against the second limit portion 212, for driving the second limit portion 212 to move or deform relative to the fixing portion 1, in such a manner that an engagement between the second limit portion 212 and the second engagement portion 12 is released. During a movement of the trigger 7, the trigger 7 does not drive the first limit portion 11 to move relative to the first engagement portion 41. That is, the trigger 7 is not used to control the first limit portion 11 and the first engagement portion 41.

With such a design, when the positional constraint on the driving unit 2 is released, the positional constraint on the mounting base 4 will not be released simultaneously. Therefore, during the implantation, a possibility of the implantation failure of the monitoring unit 3 caused by the relative movement between the mounting base 4 and the fixing portion 1 can be reduced, which is more in line with the actual use demand.

As illustrated in FIG. 1 and FIG. 2, in a possible embodiment, the inserting apparatus includes a locking member 6. The locking member 6 is configured to restrict the trigger 7 from abutting against the second limit portion 212. When the locking member 6 is in a locked state, the trigger 7 is unable to drive the second limit portion 212 to be disengaged from the second engagement portion 12. When the locking member 6 is in an unlocked state, the trigger 7 is able to abut against the second limit portion 212 to drive the second limit portion 212 to be disengaged from the second engagement portion 12.

In a possible embodiment, the inserting apparatus includes the retraction assembly 22 configured to pierce the skin. The retraction assembly 22 is relatively sharp and therefore carries a certain level of risk. With the locking member 6, a possibility of accidental triggering of the inserting apparatus can be reduced. A need to unlock the inserting apparatus before use can reduce a possibility of the retraction assembly 22 popping out due to a misoperation, which is conducive to enhancing safety of the inserting apparatus, and is more in line with the actual use demand.

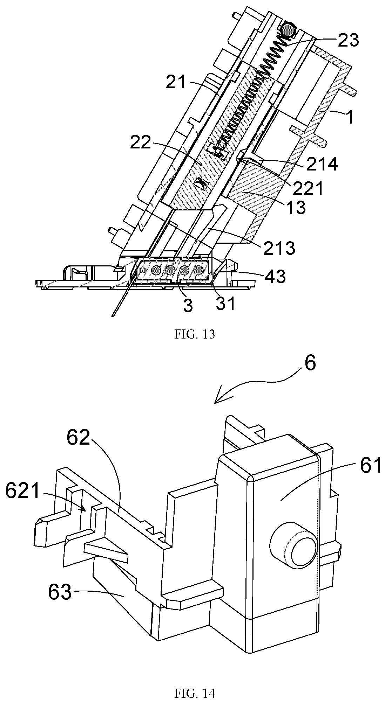

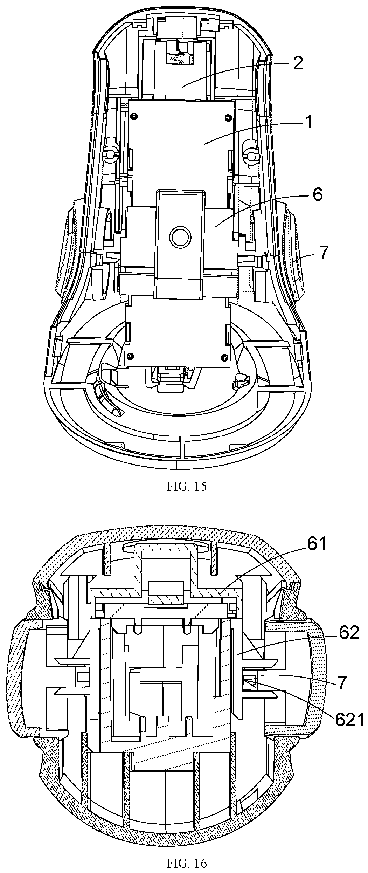

As illustrated in FIG. 14, in a possible embodiment, the locking member 6 includes a body portion 61, a first extension portion 62, and a second extension portion 63. The first extension portion 62 and the second extension portion 63 each are connected to the body portion 61. The second extension portion 63 is movable or deformable relative to the body portion 61. When the locking member 6 is in the locked state, the first extension portion 62 is located between the trigger 7 and the second limit portion 212. In this case, when pressed, the trigger 7 abuts with the first extension portion 62, and is unable to drive the second limit portion 212 to be disengaged from the second engagement portion 12. The first extension portion 62 may include a limit groove 621, as illustrated in FIG. 16. When the locking member 6 is in the locked state, at least part of the trigger 7 is located in the limit groove 621. A precision of an engagement between the first extension portion 62 and the trigger 7 can be improved through forming the limit groove 621. When the locking member 6 is in the unlocked state, the second extension portion 63 is located between the trigger 7 and the second limit portion 212. In this case, when pressed, the trigger 7 may abut with the second extension portion 63 and drive the second extension portion 63 to move or deform in a direction close to the second limit portion 212, in such a manner that the second extension portion 63 can drive the second limit portion 212 to move. In this way, the second limit portion 212 is disengaged from the second engagement portion 12 to release the positional constraint on the driving unit 2, and thus the driving unit 2 starts to perform the implantation action. Such a design can allow the second limit portion 212 to be driven by the second extension portion 63. Through transmitting a movement using the second extension portion 63, a movement distance and a size of the trigger 7 can be reduced.

As illustrated in FIG. 11 and FIG. 12, in a possible embodiment, the mounting base 4 includes a main body 42 and a fifth limit portion 43. The fifth limit portion 43 is swingable relative to the main body 42. The monitoring unit 3 includes a fifth engagement portion 31. The fifth limit portion 43 and the fifth engagement portion 31 are engaged with each other to restrict the monitoring unit 3 from detaching from the mounting base 4.

During the mounting of the monitoring unit 3 at the mounting base 4, when the monitoring unit 3 moves in the first direction, the fifth engagement portion 31 can abut with the fifth limit portion 43 and drive the fifth limit portion 43 to swing, in such a manner that the fifth limit portion 43 avoids the monitoring unit 3 during the mounting of the monitoring unit 3 to enable the monitoring unit 3 to pass over the fifth limit portion. After the mounting of the monitoring unit 3 is completed, a position of the fifth limit portion 43 can be restored, and thus the fifth limit portion 43 can be engaged with the fifth engagement portion 31 to restrict the monitoring unit 3 from leaving the mounting base 4, enhancing stability of the mounting of the monitoring unit 3.

In a possible embodiment, the monitoring unit 3 may include an electrode, but does not include at least one of a battery module, a data processing module, and a data transmission module. After the monitoring unit 3 is implanted in the human body, the needle withdrawal action is performed. After the needle is withdrawn, the fixing portion 1 can be removed, and the mounting base 4 and the monitoring unit 3 are left in the human body. In this case, an electronic unit including the battery module and the data processing module is mounted at the mounting base 4.

By separately setting up the electronic unit, a reduction of volumes of the monitoring unit 3 and the mounting base 4 can be facilitated, favorably reducing an overall volume of the inserting apparatus.

Based on the inserting apparatus according to any of the above embodiments, a method of using the inserting apparatus is further provided according to the embodiments of the present disclosure. The method is applicable in the inserting apparatus involved in any of the above embodiments and includes the following operations. The monitoring unit 3 is driven by the driving unit 2 to move from the first position relative to the fixing portion 1 in the first direction. At the first position, the first limit portion 11 of the fixing portion 1 is engaged with the first engagement portion 41 of the mounting base 4 to prevent the mounting base 4 from moving relative to the fixing portion 1. The first limit portion 11 is driven by the driving unit 2, to gradually move or deform the first limit portion 11 in a direction away from the first engagement portion 41. The driving unit 2 reaches the second position. The monitoring unit 3 is mounted to the mounting base 4. At the second position, an engagement between the first limit portion 11 and the first engagement portion 41 is at least partially released.

The driving unit 2 is configured to drive the monitoring unit 3 to move in the first direction relative to the fixing portion 1 to perform the implantation action. The retraction assembly 22 of the driving unit 2 is configured to pierce the human body to enable the electrode of the monitoring unit 3 to enter the human body along a wound, in such a manner that the glucose can be monitored. During the movement of the driving unit 2, the driving unit 2 drives the first limit portion 11 to move or deform, for releasing an engagement between the first limit portion 11 and the first engagement portion 41. When the driving unit 2 reaches the second position, the implantation of the monitoring unit 3 is completed, in which case the monitoring unit 3 is mounted at the mounting base 4, and the first limit portion 11 is at least partially disengaged from the first engagement portion 41. Since the implantation of the monitoring unit 3 is completed, a constraint on the relative position between the mounting base 4 and the fixing portion 1 can be released, facilitating a separation of the fixing portion 1 and the mounting base 4 for ease of use.

In a possible embodiment, an operation of enabling the driving unit 2 to reach the second position, mounting the monitoring unit 3 at the mounting base 4, and at least partially releasing the engagement between the first limit portion 11 and the first engagement portion 41 at the second position includes: at the second position, the first limit portion 11 and the first engagement portion 41 are completely disengaged from each other.

At the second position, the monitoring unit 3 is already mounted at the mounting base 4. In this case, the driving unit 2, the fixing portion 1, and other structures used for the implantation can be removed. By completely releasing the engagement between the first limit portion 11 and the first engagement portion 41, a resistance when removing the driving unit 2, the fixing portion 1, and other structures can be reduced to facilitate use.

In a possible embodiment, the method further includes: driving, by the trigger 7, the second limit portion 212 to move or deform, to disengage the second limit portion 212 from the second engagement portion, and enabling the driving unit 2 to drive the monitoring unit 3 to move in the first direction.

The second limit portion 212 located at the driving unit 2 and the second engagement portion 12 located at the avoidance space of the fixing portion 1 are configured to restrict a relative movement between the driving unit 2 and the fixing portion 1. The trigger 7 is configured to drive the second limit portion 212 to be disengaged from the second engagement portion 12 to release a restriction on the driving unit 2, for performing the implantation action.

In a process of the driving unit 2 driving the monitoring unit 3 to move in the first direction, the fifth engagement portion 31 drives the fifth limit portion 43 to move or deform. In response to the monitoring unit 3 reaching the second position, the fifth limit portion 43 is restored, and the fifth limit portion 43 is engaged with the fifth engagement portion 31 to prevent the monitoring unit 3 from detaching from the mounting base 4.

An engagement between the fifth limit portion 43 and the fifth engagement portion 31 is used to improve the stability of the mounting of the monitoring unit 3, which reduces a possibility of the monitoring unit 3 leaving the mounting base 4.

In a possible embodiment, the method further includes: removing the inserting apparatus and mounting an electronic unit at the mounting base 4.

In a possible embodiment, a continuous analyte monitoring apparatus is provided, which includes the aforementioned inserting apparatus and the electronic unit. The electronic unit may include at least one of the battery module, the data processing module, and the data transmission module, and may be used in conjunction with the monitoring unit 3. The battery module, the data processing module, and the data transmission module may be set without duplication in the monitoring unit 3 and the electronic unit. That is, a total of one battery module, one data processing module, and one data transmission module are included in the monitoring unit 3 and the electronic unit to reduce an overall quantity of components, facilitating the miniaturized design. The battery module may include one or more batteries. After the implantation of the monitoring unit 3 is completed, the driving unit 2 is removed, and the monitoring unit 3 for monitoring user indicators is set in the human body along with the mounting base 4. The electronic unit may be configured to provide energy, collect and/or organize data collected by the monitoring unit 3, and transmit the data to an electronic device such as a cell phone or a tablet computer via a signal such as Bluetooth or WiFi, which facilitates the user to understand his or her health status in time and is more in line with the actual use demand.

In use, the inserting apparatus is placed at a predetermined implantation position to enable the mounting base 4 to be attached to the skin at a side of the mounting base 4. The locking member 6 is unlocked. The trigger 7 is driven to move. The second extension portion 63 of the locking member 6 is driven by the trigger 7 to move towards the second limit portion 212. In this way, the second limit portion 212 is disengaged from the second engagement portion 12. The first driver 5 may be in the deformed state before the second limit portion 212 is disengaged from the second engagement portion 12. After the second limit portion 212 is disengaged from the second engagement portion 12, the restoring force of the first driver 5 can act on the insertion assembly 21 to drive the insertion assembly 21 to drive the retraction assembly 22 and the monitoring unit 3 to move in the first direction. When the driving unit 2 moves in the first direction until the abut portion 211 abuts with the connection arm 113, the driving unit 2 continues to move in the first direction. In addition, during the movement of the driving unit 2, the driving unit 2 drives the connection arm 113 to drive the first limit portion 11 to move away from the first engagement portion 41, in such a manner that the first limit segment 111 is disengaged from the first engagement portion. During the movement of the driving unit 2 in the first direction, the retraction assembly 22 can extend relative to the inserting apparatus for puncturing the skin. The electrode of the monitoring unit 3 is able to extend into the body of the user with the retraction assembly 22. When the driving unit 2 moves until the fourth limit portion 214 abuts with the fourth engagement portion 13 in the first direction, the driving unit 2 drives the monitoring unit 3 to move to the second position, in which case the monitoring unit 3 is mounted at the mounting base 4. The third limit portion 213 of the insertion assembly 21 is disengaged from the fourth engagement portion 13. The third limit portion 213 is moveable away from the third engagement portion 221 of the retraction assembly 22 to be disengaged from the third engagement portion 221, in such a manner that a positional constraint on the insertion assembly 21 and the retraction assembly 22 is released. Before the positional constraint on the insertion assembly 21 and the retraction assembly 22 is released, the second driver 23 may be in the deformed state. After the positional constraint on the insertion assembly 21 and the retraction assembly 22 is released, the restoring force of the second driver 23 can act on the retraction assembly 22 to drive the retraction assembly 22 to move in the second direction for withdrawing the needle, and the electrode of the monitoring unit 3 is left in the body of the user. After the needle is withdrawn, the user can remove the driving unit 2, the fixing portion 1, and other structures, and then the electronic unit is mounted at the mounting base 4, to facilitate a transmission of data collected by the inserting apparatus, which facilitates the user to understand his or her own physical condition in real time.

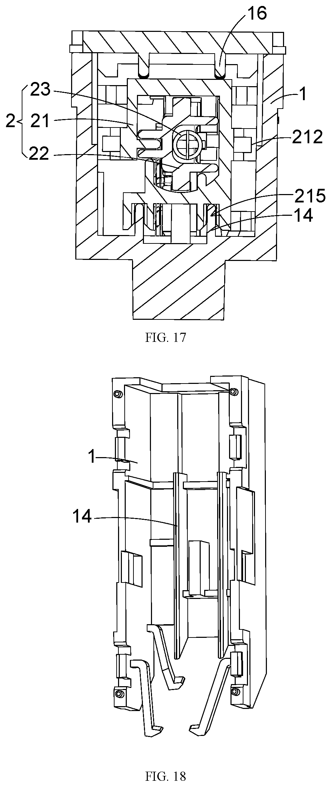

During the implantation, stability of the movement of the driving unit 2 is one of the factors affecting the success rate of the implantation of the inserting apparatus and the wound area. As illustrated in FIG. 17, in a possible embodiment, the inserting apparatus includes the fixing portion 1 and the driving unit 2. The driving unit 2 is slidably mounted in the fixing portion 1, and is movable in the first direction relative to the fixing portion 1 for performing the implantation action. The fixing portion 1 is provided with a first guide portion 14 extending in the first direction. The driving unit 2 includes a second guide portion 215 extended in the first direction and the second limit portion 212 configured to resiliently abut with the inner wall of the fixing portion 1. During a movement of the driving unit 2 relative to the fixing portion 1, the first guide portion 14 is engaged with the second guide portion 215 to guide the relative movement between the fixing portion 1 and the driving unit 2. In addition, during the movement of the driving unit 2 relative to the fixing portion 1, the second limit portion 212 is capable of resiliently abutting with the inner wall of the fixing portion 1, guiding the movement of the driving unit 2 relative to the fixing portion 1.

With the first guide portion 14 and the second guide portion 215, the stability of the movement of the driving unit 2 relative to the fixing portion 1 can be improved. The second limit portion 212 may be a resilient arm or any structure that can be resiliently deformed and is configured to abut with the inner wall of the fixing portion 1 during the movement. Since the second limit portion 212 resiliently abuts with the inner wall of the fixing portion 1, the second limit portion 212 deforms when the driving unit 2 is subjected to shaking during the movement. In this way, an interaction force between the second limit portion 212 and the fixing portion 1 is changed, which causes the driving unit 2 to move in a direction opposite to a direction of the shaking, facilitating maintaining the driving unit 2 in a stable condition. Such a design can enhance the stability of the movement of the driving unit 2 relative to the fixing portion 1, which can reduce a possibility of the driving unit 2 shaking relative to the fixing portion 1 to help reduce the wound area. Therefore, the bleeding and the pain can be alleviated, which is conducive to improving the use experience of the user.

During the implantation, when the driving unit 2 shakes due to unsatisfactory stability of the movement of the driving unit 2, the wound area in the human body is increased, which aggravates the pain and the bleeding, affecting the use experience of the user.

As illustrated in FIG. 8, FIG. 9, and FIG. 10, the second limit portion 212 includes a locked position and an unlocked position. At the locked position, the second limit portion 212 is engaged with the second engagement portion 12 of the fixing portion 1 to prevent the driving unit 2 from moving relative to the fixing portion 1. When the second limit portion 212 moves or deforms in a direction away from the second engagement portion 12, the second limit portion 212 can switch from the locked position to the unlocked position. At the unlocked position, the driving unit 2 is movable relative to the fixing portion 1.

With such a design, the second limit portion 212 can not only guide the driving unit 2 but also apply the positional constraint on the driving unit 2. The second limit portion 212 is configured to lock the driving unit 2 before the implantation action starts and guide the driving unit 2 after the implantation action has started. The second limit portion 212 can simultaneously integrate functions of positional constraint and guidance, which is conducive to reducing a quantity of components of the inserting apparatus, and can make the overall structure more compact, facilitating the miniaturized design.

As illustrated in FIG. 17 and FIG. 18, in a possible embodiment, one of the first guide portion 14 and the second guide portion 215 is a guide protrusion, and another one of the first guide portion 14 and the second guide portion 215 is a guide recess. At least part of the guide protrusion extends into the guide recess.

With such a design, a guide rail and guide groove structure can be formed to facilitate an enhancement of the stability of the movement of the driving unit 2 relative to the fixing portion 1, which is more in line with the actual use demand.

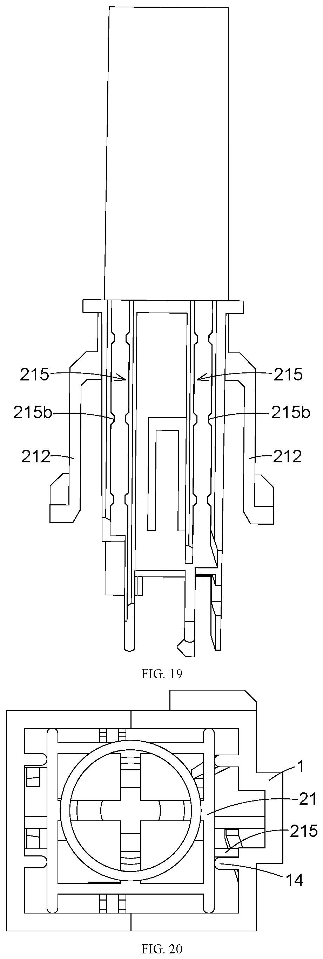

As illustrated in FIG. 19, in a possible embodiment, the guide recess is provided with at least two raised portions 215b on at least one inner side wall of the guide recess. Each of the at least two raised portions 215b protrudes towards the guide protrusion. The raised portion 215b is configured to abut with the guide protrusion when the guide protrusion is engaged with the guide recess.

With such a design, a contact area between the guide protrusion and the guide recess can be reduced, in such a manner that a friction between the first guide portion 14 and the second guide portion 215 can be reduced to reduce an effect of the friction on the relative movement between the driving unit 2 and the fixing portion 1.

As illustrated in FIG. 19, in a possible embodiment, the guide recess is provided with the raised portion 215b at an inner wall of the guide recess at each of two sides of the guide protrusion.

As illustrated in FIG. 20, in a possible embodiment, the first guide portion 14 protrudes in a direction close to the driving unit 2, and the second guide portion 215 protrudes in a direction close to the fixing portion 1. The first guide portion 14 abuts with the driving unit 2 and/or the second guide portion 215 abuts with the fixing portion 1. The first guide portion 14 may be offset from the second guide portion 215. Both the first guide portion 14 and the second guide portion 215 may be guide protrusions. With such a design, both a volume of the guide mechanism and a contact area between the first guide portion 14 and the second guide portion 215 can be reduced, favoring a reduction of the friction.

The first guide portion 14 and the second guide portion 215 may both be protrusion structures. With such a design, a reduction of the volume of the guide mechanism can be facilitated to optimize structures of the fixing portion 1 and the driving unit 2, which is conducive to reducing the volume of the inserting apparatus.

In a possible embodiment, one of surfaces on which the first guide portion 14 and the second guide portion 215 come into contact with each other is a flat surface, and another one of the surfaces on which the first guide portion 14 and the second guide portion 215 come into contact with each other is a curved surface.

With such a design, the first guide portion 14 can be in line contact with the second guide portion 215, which can not only provide guidance but also facilitate a reduction of the contact area between the first guide portion 14 and the second guide portion 215. Therefore, the friction between the first guide portion 14 and the second guide portion 215 is reduced, in such a manner that the relative movement between the driving unit 2 and the fixing portion 1 is smoother, enhancing the stability of the movement.

As illustrated in FIG. 17, in a possible embodiment, one of the fixing portion 1 and the driving unit 2 is provided with a third guide portion 16. The third guide portion 16 extends in the first direction, and protrudes in a direction close to another one of the fixing portion 1 and the driving unit 2 to abut with the other one of the fixing portion 1 and the driving unit 2.

Such a design is conducive to further improving the guidance, which therefore enhances the stability of the relative movement between the driving unit 2 and the fixing portion 1, and is more in line with the actual use demand.

As illustrated in FIG. 17, in a possible embodiment, the first guide portion 14 and the third guide portion 16 may be disposed at the fixing portion 1. The second guide portion 215 is disposed at the driving unit 2. Each of the first guide portion 14 and the third guide portion 16 may be the guide protrusion. The second guide portion 215 may be the guide recess. The first guide portion 14 and the third guide portion 16 may be disposed at a same side or different sides. The driving unit 2 may include at least two second limit portions 212 arranged at two opposite sides of the driving unit 2. The at least two second limit portions 212 at the two opposite sides may be arranged symmetrically. The first guide portion 14 and the third guide portion 16 may be arranged at two opposite sides of the driving unit 2. The first guide portion 14, the third guide portion 16, and the second guide portion 215 are not at a same side.

With such a design, the driving unit 2 can be guided from different directions, enhancing the stability of the movement of the driving unit 2.

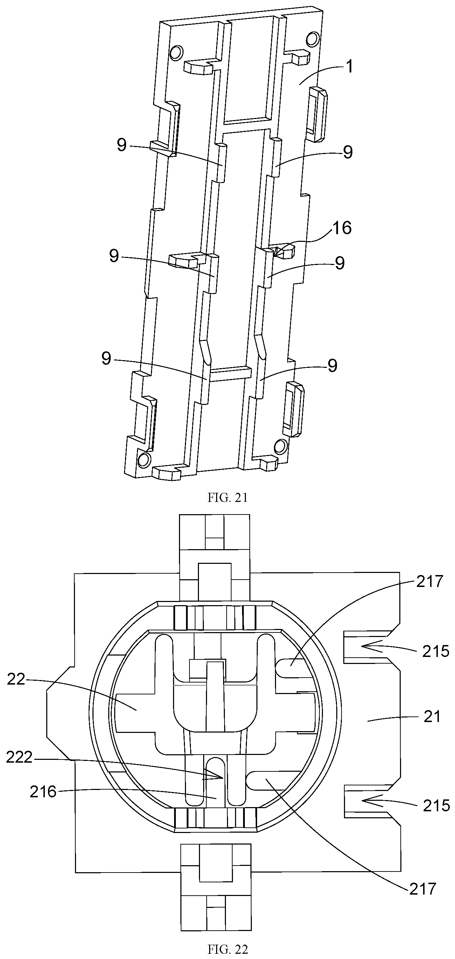

As illustrated in FIG. 17, in a possible embodiment, each of the first guide portion 14 and the third guide portion 16 is the guide protrusion and includes at least two guide segments 9. The guide segments 9 included in the first guide portion 14 and the third guide portion 16 are arranged at intervals in the first direction. A structure of the third guide portion 16 is illustrated in FIG. 21. The first guide portion 14 may have a structure similar to that of the third guide portion 16 and also include a plurality of guide segments 9 arranged at intervals.

Each of the first guide portion 14 and the third guide portion 16 may include a plurality of protrusion structures. With such a design, a volume of the guide protrusion can be reduced, which facilitates a reduction of a contact area between the driving unit 2 and the fixing portion 1 to reduce friction between the driving unit 2 and the fixing portion 1. Therefore, an impact exerted by the friction on the relative movement between the driving unit 2 and the fixing portion 1 is lowered, which is conducive to enhancing the stability of the movement.

As illustrated in FIG. 22, in a possible embodiment, the insertion assembly 21 includes a fourth guide portion 216 extending in the second direction, and the retraction assembly 22 includes a fifth guide portion 222 extending in the second direction. The fourth guide portion 216 is engaged with the fifth guide portion 222 for guiding a movement of the retraction assembly 22.

With such a design, the stability of the movement of the retraction assembly 22 relative to the insertion assembly 21 when withdrawing the needle can be enhanced, contributing to reducing a possibility of shaking of the retraction assembly 22 in a process of withdrawing the needle. Therefore, the wound area can be reduced to alleviate the bleeding.

One of the fourth guide portion 216 and the fifth guide portion 222 is the guide protrusion, and another one of the fourth guide portion 216 and the fifth guide portion 222 is the guide recess. At least part of the guide protrusion extends into the guide recess.

With such a design, an engagement between the guide rail and the guide groove can be formed, which is conducive to enhancing the stability of the movement of the retraction assembly 22 relative to the insertion assembly 21.

In a possible embodiment, in the first direction, the fixing portion 1 has a size of c, a movement distance of the retraction assembly 22 in the second direction is d, and each of the fourth guide portion 216 and the fifth guide portion 222 has a size of e, where e≥40% c and/or e≥2d.

With such a design, a guide distance for the retraction assembly 22 can exceed 40% of a size of the fixing portion 1 and exceed twice a movement distance at which the needle is withdrawn, which is conducive to enhancing the stability of the movement when withdrawing the needle.

As illustrated in FIG. 22, in a possible embodiment, one of the insertion assembly 21 and the retraction assembly 22 is provided with a sixth guide portion 217. The sixth guide portion 217 extends in the second direction. The sixth guide portion 217 protrudes towards and abuts with another one of the insertion assembly 21 and the retraction assembly 22.

With such a design, the movement between the insertion assembly 21 and the retraction assembly 22 can be guided to enhance the stability of the movement of the retraction assembly 22 when withdrawing the needle, which reduces a possibility of an increase in the wound area caused by the shaking of the retraction assembly 22 in the process of withdrawing the needle.

The sixth guide portion 217 may be disposed at the insertion assembly 21. The fourth guide portion 216 and the sixth guide portion 217 may be located at different sides of the insertion assembly 21. In this way, the fourth guide portion 216 and the sixth guide portion 217 can be engaged with the retraction assembly 22 from different directions, which is conducive to enhancing the stability of the movement of the retraction assembly 22 and is more in line with the actual use demand.

In a possible embodiment, the fourth guide portion 216 and the sixth guide portion 217 may be guide protrusions. The fourth guide portion 216 may include at least two guide segments 9. The at least two guide segments 9 included in the fourth guide portion 216 are spaced apart from each other in the second direction. The sixth guide portion 217 may include at least two guide segments 9. The at least two guide segments 9 included in the sixth guide portion 217 are spaced apart from each other in the second direction. Each of the fourth guide portion 216 and the sixth guide portion 217 may have a structure similar to that of the third guide portion 16 and be provided with a plurality of protrusion structures arranged at intervals.

While some embodiments of the present disclosure have been described above, the present disclosure is not limited to these embodiments. It is conceivable for those skilled in the art that various changes and variations can be made to the present disclosure. Any modifications, equivalent replacements, improvements, or the like made within the spirit and the principle of the present disclosure shall equally fall within the scope of the present disclosure.

## Claims

1. An inserting apparatus for subcutaneously implantable biosensor placement, comprising:
a housing;
a mounting base adapted to be attached to skin of a host;
a fixing portion disposed in the housing and removably engaged with the mounting base;
a driving unit movably disposed in the fixing portion;
a monitoring unit detachably connected to the driving unit, the driving unit being movable relative to the fixing portion to drive the monitoring unit to be mounted at the mounting base; and
a limiting unit configured to prevent the fixing portion from moving relative to the housing during mounting of the monitoring unit at the mounting base.

2. The inserting apparatus according to claim 1, wherein:
the limiting unit comprises a first limit portion located on the fixing portion and a first engagement portion located on the mounting base, the first engagement portion being engaged with the first limit portion to prevent the fixing portion from moving relative to the mounting base.

3. The inserting apparatus according to claim 2, wherein:
the driving unit is configured to move in a first direction to drive the monitoring unit to move from a first position to a second position, wherein the monitoring unit is mounted at the mounting base at the second position; and
before the monitoring unit reaches the second position, the first limit portion is configured to be engaged with the first engagement portion to limit the relative movement between the fixing portion and the mounting base.

4. The inserting apparatus according to claim 3, wherein at the second position, a force between the first limit portion and the first engagement portion is smaller than an adhesion force between the mounting base and the skin.

5. The inserting apparatus according to claim 3 or 4, wherein at the second position, at least part of the first limit portion is disengaged from the first engagement portion to allow the fixing portion to move relative to the mounting base.

6. The inserting apparatus according to claim 3 or 4 or 5, wherein:
the fixing portion has a mounting wall and a connection arm connected between the mounting wall and the first limit portion, the driving unit comprises an abut portion;
at the first position, the abut portion being spaced apart from the connection arm, when the driving unit moves in the first direction, the abut portion being capable of abutting with the connection arm to drive the connection arm to move or deform in a direction gradually away from the first engagement portion.

7. The inserting apparatus according to claim 6, wherein an acute angle between the connection arm and the first direction ranges from 15° to 45°.

8. The inserting apparatus according to any one of the claims 3 to 7, wherein:
the driving unit comprises an insertion assembly and a first driver, the insertion assembly comprising at least one second limit portion; and
the fixing portion comprises at least one second engagement portion,
wherein the at least one second limit portion is engaged with the at least one second engagement portion to restrict a movement of the insertion assembly relative to the fixing portion; and
wherein the at least one second limit portion being configured to be moveable or deformable relative to the fixing portion to allow the driving unit to move relative to the fixing portion in the first direction.

9. The inserting apparatus according to claim 8, further comprising a trigger, the trigger being adapted to abut against the second limit portion to make the second limit portion move or deform relative to the fixing portion, releasing an engagement between the second limit portion and the second engagement portion.

10. The inserting apparatus according to claim 9, further comprising a locking member for preventing the trigger from abutting against the second limit portion.

11. The inserting apparatus according to any one of claims 1 to 10, wherein:
the mounting base comprises a main body and a fifth limit portion, the fifth limit portion being swingable relative to the main body; and
the monitoring unit comprises a fifth engagement portion,
wherein the fifth limit portion is engaged with the fifth engagement portion to restrict the monitoring unit from detaching from the mounting base.

12. A method for using an inserting apparatus for subcutaneously implantable biosensor placement, wherein the inserting apparatus comprises a fixing portion, a driving unit, a monitoring unit, and a mounting base, the method comprising:
driving, by the driving unit, the monitoring unit to move from a first position relative to the fixing portion in a first direction, wherein at the first position, a first limit portion of the fixing portion is engaged with a first engagement portion of the mounting base to prevent the mounting base from moving relative to the fixing portion;
driving, by the driving unit, the first limit portion to gradually move or deform in a direction away from the first engagement portion; and
moving the driving unit to a second position, and mounting the monitoring unit to the mounting base, wherein at the second position, an engagement between the first limit portion and the first engagement portion is at least partially released.

13. The method according to claim 12, wherein at the second position, the first limit portion and the first engagement portion are completely disengaged from each other.

14. The method according to claim 13, wherein the inserting apparatus further comprises a trigger, the driving unit comprises a second limit portion, and the fixing portion comprises an avoidance space and a second engagement portion disposed in the avoidance space, the method further comprising:
driving, by the trigger, the second limit portion to move or deform to disengage the second limit portion from the second engagement portion, enabling the driving unit to drive the monitoring unit to move in the first direction.

15. The method according to claim 14, wherein the mounting base comprises a fifth limit portion, and the monitoring unit comprises a fifth engagement portion, the method further comprising:
driving, by the fifth engagement portion, the fifth limit portion to move or deform in a process of the driving unit driving the monitoring unit to move in the first direction; and
restoring, in response to the monitoring unit reaching the second position, the fifth limit portion, and engaging the fifth limit portion with the fifth engagement portion to prevent the monitoring unit from detaching from the mounting base.
